# EUROPÄISCHE PATENTSCHRIFT

(11) **EP 1 677 693 B1**
(45) Veröffentlichungstag und Bekanntmachung des Hinweises auf die Patenterteilung: **15.04.2009**
(21) Anmeldenummer: 03753216.5
(22) Anmeldetag: 30.10.2003
(51) Int. Cl.: A61B 17/80, A61B 17/72

(54) **KNOCHENPLATTE**
BONE PLATE
PLAQUE D'OSTEOSYNTHESE

(43) Veröffentlichungstag der Anmeldung: 12.07.2006
(73) Patentinhaber: Synthes GmbH, 4436 Oberdorf (CH)
(72) Erfinder: BEUTTER, Florian, CH-4500 Solothurn (CH); APPENZELLER, Andreas, CH-2504 Biel (CH); CICOIRA, Franco, CH-2545 Selzach (CH); FRIGG, Robert, CH-2544 Bettlach (CH)
(74) Vertreter: Lusuardi, Werther
(86) Internationale Anmeldenummer: PCT/CH2003/000712
(87) Internationale Veröffentlichungsnummer: WO 2005/041796

(56) Entgegenhaltungen:
- WO-A-01/54601
- WO-A-96/29948
- WO-A-02/096309
- DE-U- 20 309 361
- US-A- 6 030 389

## Beschreibung

Die Erfindung bezieht sich auf eine Knochenplatte gemäss dem Oberbegriff des Patentanspruchs 1.

Diese Knochenplatte ist in erster Linie dazu gedacht Vorfussosteotomien, insbesondere distale Hallux Valgus Osteotomien zu fixieren. Solche Osteotomien, bzw. deren Fixation sollten nach folgenden Kriterien erfolgen:
- Rotationsstabil,
- Passend der entsprechenden Stärke der Korrektur,
- Minimalinvasiv

Aus der WO00/06036 und der Gebrauchsmusterschrift AT 000937U2 ist ein Implantat in Form einer Spange bekannt, welche intramedullär angewandt wird. Diese hat nur den Vorteil der minimalen Invasivität bei einer intramedullären Fixierung; vom Implantat selbst geht aber keine Rotationsstabilität aus. Bei der Korrektur ist man abhängig von den verschiedenen Kröpfungen mit welchen die Spange angeboten wird. Die Spange ist somit minimal invasiv, kann jedoch keine kleinen Korrekturen fixieren.

Die WO01/54601 FRIGG offenbart eine Knochenplatte mit einem Kombinationsloch, d.h. zwei sich durchdringenden Plattenbohrungen. Nachteilig bei diesem Kombinationsloch ist der Umstand, dass nur eine der beiden Plattenbohrungen ein partielles Innengewinde trägt, so dass nur in dieser einen Plattenbohrung eine winkelstabile, rigide Verankerung einer Knochenschraube möglich ist. Der Oberbegriff des Anspruchs 1 basiert auf diese Offenbarung.

Hier will die Erfindung Abhilfe schaffen. Der Erfindung liegt die Aufgabe zugrunde, eine sowohl intramedullär wie extramedulläre anwendbare Knochenplatte zu schaffen, mit weicher eine rotationsstabile und minimal invasive Fixation kleiner sowie grosser Korrekturen möglich ist.

Die Erfindung löst die gestellte Aufgabe mit einer Knochenplatte, welche die Merkmale des Anspruchs 1 aufweist. Ausführungen dieser Knochenplatte können alle oben angeführten Anforderungen wie folgt erfüllen:
a) Rotationsstabilität:
   Durch zwei Löcher im Schaft und zwei im Kopfbereich des Metatarsale ist die Knochenplatte 1 beiderseits ausreichend rotationsstabil. Durch zwei zylindrischen Gewinde innerhalb der Platte im Schaftbereich, und Verriegelung dieser Platte mit zwei Schrauben ist die Platte auch gegen das Verdrehen sowie das Verschieben innerhalb der Markraumhöhle geschützt.
b) Fixierung passend der entsprechenden Stärke der Korrektur:
   Dies wird mit Hilfe des Zielbügels erfüllt. Das MT- Köpfchen wird je nach Wunsch entsprechend stark nach Lateral verschoben. Entlang des Köpfchens wird dann die Platte eingeführt bzw. eingeschlagen und anschliessend verschraubt.
c) Minimal invasiv:
   Der Sägeschnitt wird durch eine Inzision vorgenommen, welche gerade gross genug ist, dass das Sägeblatt hindurch passt. Diese Inzision reicht jedoch aus, die Platte einzuführen. Die Löcher zur Verschraubung der Platte im Schaftbereich können dann durch die Haut gebohrt werden.
d) Auch kleine Korrekturen möglich:
   Kleine Korrekturen, welche nicht über den kortikalen Rand des Markraumes hinausreichen, können mit dieser Platte extramedullär fixiert werden. Hierbei wird die Platte leicht entsprechend der Korrektur angebogen und unter die Haut geschoben. Der zweite Teil der Löcher, welche zur Verriegelung des Schaftes gedacht sind, sind mit einem konischen Gewinde versehen und zur extramedullären winkelstabilen Fixation gedacht. Die Löcher können, wenn die Platte unter die Haut geschoben wird, ertastet werden. Ein Einschnitt zwischen den beiden genüge dann, um beide Löcher zu erreichen.

Mit Hilfe des "Kombiloches" der erfindungsgemässen Knochenplatte - von welchem vorzugsweise zwei Stück im unteren, proximalen Teil der Knochenplatte angebracht sind - ist es möglich die Knochenplatte sowohl intramedullär, als auch extramedullär zu verwenden.
Das "Kombiloch" besteht aus einem zylindrischen und einem konischen, jeweils mit einem - mindestens partiellen - Gewinde versehenen Teil. Der zylindrische Teil findet bei einer intramedullären Fixation Anwendung und wird mit einem Schaftgewinde einer Knochenschraube verriegelt. Der konische Teil wird bei einer extramedullären Anwendung der Knochenplatte ebenfalls zur Verriegelung der Knochenplatte verwendet, wobei in diesem Fall eine Gewindekopf-Schraube verwendet wird, so dass ein sogenannter "fixateur interne" resultiert. Da das Metatarsale Köpfchen verschoben wird, ist hier eine Auswahl der Fixierung (intramedullär/extramedullär) nicht von Bedeutung. Daher entsprechen die beiden Plattenlöcher im oberen, distalen Teil der Knochenplatte den üblichen winkelstabilen Plattenlöchern (kreiszylindrisches oder kegelförmiges Loch mit Innengewinde).

Zur intramedullären Fixation der erfindungsgemässen Knochenplatte ist ein Zielgerät notwendig, welches es möglich macht, die Knochenplatte präzise im Knochen zu verriegeln. Dieses Zielgerät soll aber gleichzeitig eine genaue Plazierung der Knochenplatte ermöglichen und als Einschlaginstrument dienen.

Bei der extramedullären Fixation ist kein Zielgerät notwendig, da die Löcher der Knochenplatte unter der Haut ertastet werden können. Die beiden, vorzugsweise am proximalen Ende der Knochenplatte angeordneten "Kombilöcher" liegen so nahe beieinander, dass sie durch eine Inzision und Verschieben der Haut erreicht werden können.

Als Innengewinde sollen nicht nur helixförmige Strukturen verstanden werden, sondern auch rippenförmige Strukturen, welche die gleiche Funktion wie ein Gewinde erfüllen können.

Die Knochenplatte wie sie im Anspruch 1 definiert ist hat den Vorteil, dass bei der Herstellung ein geringerer Materialverlust entsteht. Ein zweiter Vorteil besteht darin, dass eine Verkürzung der Knochenplatte möglich ist, was eine weniger invasive Wirkung zur Folge hat.

Bei einer besonderen Ausführungsform ist das aus zwei sich überlappenden Bohrungen bestehende Plattenloch nahe beim ersten Ende der Knochenplatte angebracht.

Bei einer bevorzugten Ausführungsform verjüngt sich das erste Ende der Knochenplatte. Dadurch lässt sie sich leichter in die Knochenhöhle einführen und - bei der extramedullären Anwendung - leichter unter die Haut schieben.

Bei einer weiteren Ausführungsform ist das Innengewinde der ersten Bohrung mehrgängig, vorzugsweise zweigängig ausgebildet. Dadurch erfolgt ein rascheres Fassen beim Eindrehen einer Gewindekopf-Schraube. Auch das Innengewinde der zweiten Bohrung kann mehrgängig, vorzugsweise zweigängig ausgebildet sein.

Bei einer weiteren Ausführungsform verlaufen die Zylinderachse und die Kegelachse der beiden sich überlappenden Bohrungen im wesentlichen parallel zueinander. Vorzugsweise weisen die Zylinderachse und die Kegelachse einen Abstand A > 0,1 mm voneinander auf.

Bei einer besonderen Ausführungsform besitzt die Knochenplatte mindestens zwei Plattenlöcher, welche aus einer Kombination von zwei verschiedenartigen, sich teilweise überlappenden Bohrungen gebildet werden. Vorzugsweise schliesst sich daran mindestens ein weiteres Plattenloch an, welches nicht aus zwei sich teilweise überlappenden Bohrungen gebildet wird. Die an die Kombilöcher anschliessenden Plattenlöcher können zylindrisch oder konisch, mit oder ohne Innengewinde ausgebildet sein.

Bei einer weiteren Ausführungsform weist die Knochenplatte ein Kompressionsloch auf.

Bei einer weiteren Ausführungsform ist das zweite Ende der Knochenplatte Y-förmig ausgebildet. Das zu fixierende Fragment ist eventuell zu kurz, um mit zwei hintereinander liegenden Löchern fixiert zu werden. In diesem Fall bietet ein Y-förmiges Ende der Knochenplatte den Vorteil, dass sich jeweils in einem der Y-Schenkel ein Loch befindet.
Vorzugsweise weist dabei einer (oder auch beide) der beiden Äste des Y-förmigen Endes ein Kompressionsloch auf. Es kann auch ein Kombinationsloch verwendet werden, d.h. eine Kombination eines Kompressions- mit einem Verriegelungsloch. Es kann aber auch nur eines der beiden Typen von Löchern (Kompressionsloch oder Verriegelungsloch) verwendet werden.
Ein Kompressionsloch in Kombination mit einem Verriegelungsloch am anderen Arm des "Y" hat den Vorteil, dass man über die Knochenplatte komprimieren kann, bevor man über das zweite Loch winkelstabil fixiert.

Bei einer weiteren Ausführungsform ist die Oberseite und die Unterseite der Knochenplatte gekrümmt ausgebildet. Die gekrümmte Oberseite und Unterseite entsprechen typischerweise im wesentlichen den Oberflächen zweier Kreiszylinder K_{Oberseite} und K_{Unterseite}.
Vorzugsweise sind die Oberseite (2) und die Unterseite (3) unterschiedlich stark gekrümmt. Wenn die Platte sowohl intramedullär wie extramedulläre verwendet wird, kann mit der unterschiedlichen Krümmung der Ober- und Unterseite eine optimale Anpassung an die intramedulläre Oberfläche des Markraums einerseits und an die extramedulläre Oberfläche des Knochens anderseits erzielt werden.
Bei einer besonderen Ausführungsform beträgt der Radius R_{Oberseite} des Kreiszylinders K_{Oberseite} höchstens 50 %, vorzugsweise höchstens 40 % des Radius R_{Unterseite} des Kreiszylinders K_{Unterseite}.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine Aufsicht auf die Knochenplatte gemäss der Erfindung mit zwei Kombinationslöchern; und
Fig. 2 einen Längsschnitt durch die Knochenplatte nach Fig. 1 im Bereich eines der Kombinationslöcher.

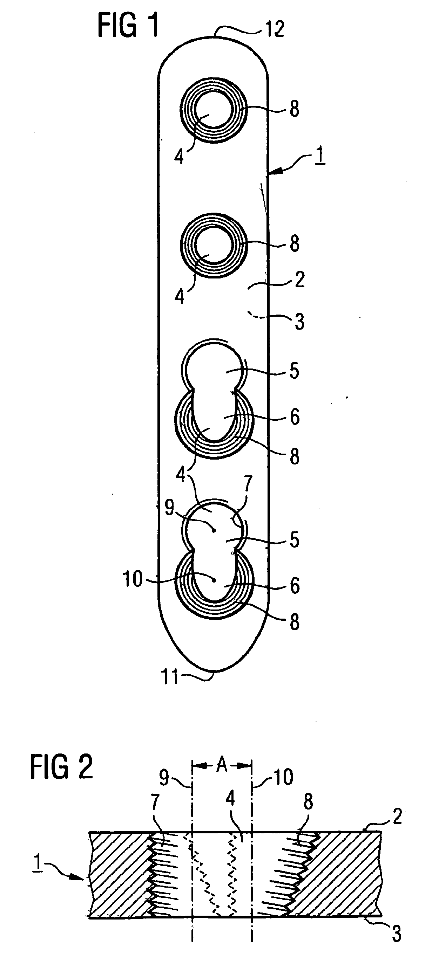

Die in den Fig. 1 und 2 dargestellte Knochenplatte 1 besitzt eine Oberseite 2, eine knochenseitige Unterseite 3, ein erstes Ende 11, ein zweites Ende 12 und vier zwischen den beiden Enden 11,12 angeordnete, die Oberseite 2 mit der Unterseite 3 verbindende Plattenlöcher 4 zur Aufnahme von Knochenschrauben.
Die beiden dem ersten Ende 11 benachbarten Plattenlöcher 4 sind aus einer Kombination von zwei verschiedenartigen, sich teilweise überlappenden Bohrungen 5,6 gebildet. Dabei ist die erste der beiden Bohrungen 5 kreiszylindrisch ausgebildet mit einer Zylinderachse 9 und einem Innengewinde 7. Die zweite der beiden Bohrungen 6 verjüngt sich von der Oberseite 2 nach der Unterseite 3 hin kegelstumpfförmig und besitzt eine Kegelachse 10 sowie ein Innengewinde 8. Die Zylinderachse 9 und die Kegelachse 10 verlaufen parallel zueinander und weisen voneinander einen Abstand A von 2 mm auf.
Das endständige der beiden aus zwei sich überlappenden Bohrungen 5,6 bestehenden Plattenlöcher 4 ist nahe beim ersten Ende 11 der Knochenplatte 1 angebracht, welches sich verjüngt.
Die an die Kombilöcher anschliessenden Plattenlöcher 4 sind konisch und winkelstabil mit einem Innengewinde 8 ausgebildet.

## Patentansprüche

1. Knochenplatte (1) mit einer Oberseite (2), einer knochenseitigen Unterseite (3), einem ersten Ende (11), einem zweiten Ende (12) und mehreren, zwischen den beiden Enden (11,12) angeordnete, die Oberseite (2) mit der Unterseite (3) verbindenden Plattenlöcher (4) zur Aufnahme von Knochenschrauben, wobei mindesten eines der Plattenlöcher (4) aus einer Kombination von zwei verschiedenartigen, sich teilweise überlappenden Bohrungen (5,6) gebildet wird und die erste der beiden Bohrungen (5) kreiszylindrisch ausgebildet ist, mindestens teilweise ein Innengewinde (7) trägt und eine Zylinderachse (9) aufweist,
**dadurch gekennzeichnet, dass**
A) die zweite der beiden Bohrungen (6) eine Kegelachse (10) aufweist und sich von der Oberseite (2) nach der Unterseite (3) hin kegelstumpfförmig verjüngt;
B) die zweite der beiden Bohrungen (6) mindestens teilweise ein Innengewinde (8) trägt; und
C) die Zylinderachse (9) einen von Null verschiedenen Abstand A zur Kegelachse (10) besitzt.

2. Knochenplatte (1) nach Anspruch 1, **dadurch gekennzeichnet, dass** das aus zwei sich überlappenden Bohrungen (5,6) bestehende Plattenloch (4) nahe beim ersten Ende (11) der Knochenplatte (1) angebracht ist.

3. Knochenplatte (1) nach Anspruch 1 oder 2, **dadurch gekennzeichnet, dass** sich das erste Ende (11) der Knochenplatte (1) verjüngt.

4. Knochenplatte (1) nach einem der Ansprüche 1 bis 3, **dadurch gekennzeichnet, dass** das Innengewinde (7) der ersten Bohrung (5) mehrgängig, vorzugsweise zweigängig ausgebildet ist.

5. Knochenplatte (1) nach einem der Ansprüche 1 bis 4, **dadurch gekennzeichnet, dass** das Innengewinde (8) der zweiten Bohrung (6) mehrgängig, vorzugsweise zweigängig ausgebildet ist.

6. Knochenplatte (1) nach einem der Ansprüche 1 bis 5, **dadurch gekennzeichnet, dass** die Zylinderachse (9) und die Kegelachse (10) im wesentlichen parallel zueinander verlaufen.

7. Knochenplatte (1) nach einem der Ansprüche 1 bis 6, **dadurch gekennzeichnet, dass** die Zylinderachse (9) und die Kegelachse (10) einen Abstand A > 0,1 mm voneinander aufweisen.

8. Knochenplatte (1) nach einem der Ansprüche 1 bis 7, **dadurch gekennzeichnet, dass** mindestens zwei der Plattenlöcher (4) aus einer Kombination von zwei verschiedenartigen, sich teilweise überlappenden Bohrungen (5,6) gebildet werden.

9. Knochenplatte (1) nach Anspruch 8, **dadurch gekennzeichnet, dass** sie zusätzlich mindestens ein weiteres Plattenloch (4) besitzt, welches nicht aus zwei sich teilweise überlappenden Bohrungen (5,6) gebildet wird.

10. Knochenplatte (1) nach einem der Ansprüche 1 bis 9, **dadurch gekennzeichnet, dass** das zweite Ende (12) Y-förmig ausgebildet ist.

11. Knochenplatte (1) nach einem der Ansprüche 1 bis 10, **dadurch gekennzeichnet**, sie ein Kompressionsloch aufweist.

12. Knochenplatte nach Anspruch 11, **dadurch gekennzeichnet, dass** einer der beiden Äste des Y-förmigen Endes (12) ein Kompressionsloch aufweist.

13. Knochenplatte (1) nach einem der Ansprüche 1 bis 12, **dadurch gekennzeichnet, dass** die Oberseite (2) und die Unterseite (3) gekrümmt sind.

14. Knochenplatte (1) nach Anspruch 13, **dadurch gekennzeichnet, dass** die gekrümmte Oberseite (2) und Unterseite (3) im wesentlichen den Oberflächen zweier Kreiszylinder K_{Oberseite} und K_{Unterseite} entsprechen.

15. Knochenplatte (1) nach Anspruch 13 oder 14, **dadurch gekennzeichnet, dass** die Oberseite (2) und die Unterseite (3) unterschiedlich stark gekrümmt sind.

16. Knochenplatte (1) nach Anspruch 15, **dadurch gekennzeichnet, dass** der Radius R_{Oberseite} des Kreiszylinders K_{Oberseite} höchstens 50 %, vorzugsweise höchstens 40 % des Radius R_{Unterseite} des Kreiszylinders K_{Unterseite} beiträgt.

## Claims

1. A bone plate (1) comprising a top side (2), a bottom side (3) facing the bone, a first end (11), a second end (12) and several plate holes (4) which receive bone screws and are configured between the two ends (11, 12) and connect the top side (2) to the bottom side (3), at least one of said plate holes (4) being constituted by a combination of two different and partially mutually overlapping boreholes (5, 6), the first (5) of said boreholes being circular cylindrical and fitted at least partially with an inside thread (7) and subtending a cylinder axis (9),
**characterized in that**
(a) the second (6) of said boreholes subtending a cone axis (10) and tapering from the top side (2) toward the bottom side (3) in the manner of a frustrum of cone,
(b) the second (6) of said boreholes being fitted at least partially with an inside thread (8), and
(c) the cylinder axis (9) being a distance A different from zero from the cone axis (10).

2. Bone plate as claimed in claim 1, **characterized in that** the plate hole (4) consisting of two mutually overlapping boreholes (5, 6) is configured near the first end (11) of the bone plate (1).

3. Bone plate (1) as claimed in either of claims 1 and 2, **characterized by** a tapering first end (11) of the bone plate (1).

4. Bone plate as claimed in one of claims 1 through 3, **characterized in that** the inside thread (7) of the first borehole (5) is multiple threaded, preferably double threaded.

5. Bone plate (1) as claimed in one of claims 1 through 4, **characterized in that** the inside thread (8) of the second borehole (6) is multiple threaded, preferably double threaded.

6. Bone plate as claimed in one of claims 1 through 5, **characterized in that** the cylinder axis (9) and the cone axis (10) run substantially parallel to each other.

7. Bone plate (1) as claimed in one of claims 1 through 6, **characterized in that** the cylinder axis (9) and the cone axis (10) are a distance A > 0.1 mm from each other.

8. Bone plate (1) as claimed in one of claim 1 through 7, **characterized in that** at least two of the plate holes (4) are constituted by a combination of two different and partially mutually overlapping boreholes (5, 6).

9. Bone plate (1) as claimed in claim 8, **characterized in that** it comprises additionally at least one further plate hole (4) which does not consist of two partly overlapping boreholes (5, 6).

10. Bone plate (1) as claimed in one of claims 1 through 9, **characterized in that** the second end (12) assumes a Y shape.

11. Bone plate (1) as claimed in one of claims 1 through 10, **characterized in that** it comprises a compression hole.

12. Bone plate as claimed in claim 11, **characterized in that** one of the two arms of the Y-shaped end (12) comprises a compression hole.

13. Bone plate (1) as claimed in one of claims 1 through 12, **characterized in that** the top side (2) and the bottom side (3) are curved.

14. Bone plate (1) as claimed in claim 13, **characterized in that** the curved top side (2) the curved bottom side (3) substantially correspond to the surfaces of two circular cylinders C_{topside} and C_{bottomside}.

15. Bone plate (1) as claimed in either of claims 13 and 14, **characterized in that** the top side (2) and the bottom side (3) exhibit different curvatures.

16. Bone plate (1) as claimed in claim 15, **characterized in that** the radius R_{topside} of the circular cylinder C_{topside} is at most 50 %, preferably at most 40 % of the radius R_{bottomside} of the circular cylinder C_{bottom} side.

## Revendications

1. Plaque d'ostéosynthèse (1) présentant une face supérieure (2), une face inférieure (3) du côté de l'os, une première extrémité (11), une deuxième extrémité (12) et plusieurs alésages de plaque (4) disposés entre les deux extrémités (11, 12) reliant la face supérieure (2) à la face inférieure (3) destinés à loger des vis à os, dans laquelle au moins un des alésages de plaque (4) est formé par une combinaison de deux alésages différents se recouvrant partiellement (5, 6) et le premier des deux alésages (5) est réalisé en forme de cylindre circulaire, est pourvu au moins en partie d'un taraudage (7) et présente un axe de cylindre (9),
**caractérisée en ce que**
A) le deuxième des deux alésages (6) présente un axe de cône (10) et se rétrécit de manière conique de la face supérieure (2) vers la face inférieure (3) ;
B) le deuxième des deux alésages (6) est pourvu au moins en partie d'un taraudage (8) ; et
C) l'axe de cylindre (9) présente une distance A différente de zéro par rapport à l'axe de cône (10).

2. Plaque d'ostéosynthèse (1) selon la revendication 1, **caractérisée en ce que** l'alésage de plaque (4) formé de deux alésages se recouvrant partiellement (5, 6) est disposé à proximité de la première extrémité (11) de la plaque d'ostéosynthèse (1).

3. Plaque d'ostéosynthèse (1) selon la revendication 1 ou 2, **caractérisée en ce que** la première extrémité (11) de la plaque d'ostéosynthèse (1) se rétrécit.

4. Plaque d'ostéosynthèse (1) selon l'une quelconque des revendications 1 à 3, **caractérisée en ce que** le taraudage (7) du premier alésage (5) est à filetage multiple, de préférence à filetage double.

5. Plaque d'ostéosynthèse (1) selon l'une quelconque des revendications 1 à 4, **caractérisée en ce que** le taraudage (8) du deuxième alésage (6) est à filetage multiple, de préférence à filetage double.

6. Plaque d'ostéosynthèse (1) selon l'une quelconque des revendications 1 à 5, **caractérisée en ce que** l'axe de cylindre (9) et l'axe de cône (10) s'étendent essentiellement parallèlement l'un à l'autre.

7. Plaque d'ostéosynthèse (1) selon l'une quelconque des revendications 1 à 6, **caractérisée en ce que** l'axe de cylindre (9) et l'axe de cône (10) présentent une distance A > 0,1 mm l'un par rapport à l'autre.

8. Plaque d'ostéosynthèse (1) selon l'une quelconque des revendications 1 à 7, **caractérisée en ce qu'**au moins deux des alésages de plaque (4) sont formés à partir d'une combinaison de deux alésages différents se recouvrant partiellement (5, 6).

9. Plaque d'ostéosynthèse (1) selon la revendication 8, **caractérisée en ce qu'**elle présente en plus au moins un autre alésage de plaque (4), lequel n'est pas formé par deux alésages se recouvrant partiellement (5, 6).

10. Plaque d'ostéosynthèse (1) selon l'une quelconque des revendications 1 à 9, **caractérisée en ce que** la deuxième extrémité (12) est réalisée en forme de Y.

11. Plaque d'ostéosynthèse (1) selon l'une quelconque des revendications 1 à 10, **caractérisée en ce qu'**elle présente un trou de compression.

12. Plaque d'ostéosynthèse selon la revendication 11, **caractérisée en ce que** l'une des deux branches de l'extrémité en forme de Y (12) présente un trou de compression.

13. Plaque d'ostéosynthèse (1) selon l'une quelconque des revendications 1 à 12, **caractérisée en ce que** la face supérieure (2) et la face inférieure (3) sont courbées.

14. Plaque d'ostéosynthèse (1) selon la revendication 13, **caractérisée en ce que** la face supérieure courbée (2) et la face inférieure courbée (3) correspondent essentiellement aux surfaces de deux cylindres circulaires K_{face} supérieure et K_{face} inférieure

15. Plaque d'ostéosynthèse (1) selon la revendication 13 ou 14, **caractérisée en ce que** la face supérieure (2) et la face inférieure (3) présentent une courbure différente.

16. Plaque d'ostéosynthèse (1) selon la revendication 15, **caractérisée en ce que** le rayon R_{face} supérieure du cylindre circulaire K_{face} supérieure correspond au maximum à 50%, de préférence au maximum à 40% du rayon R_{face} inférieure du cylindre circulaire K_{face} inférieure.
